# EUROPEAN PATENT APPLICATION

(11) **EP 2 298 201 A1**
(43) Date of publication of application: **23.03.2011**
(21) Application number: 09169027.1
(22) Date of filing: 31.08.2009
(51) Int. Cl.: A61B 17/72, A61B 17/74

(54) **Arrangement for internal bone support**

(71) Applicant: Ozics Oy, 33520 Tampere (FI)
(72) Inventor: Kaikkonen, Auvo, SLM1601, Sliema (MT); Pösel, Andreas, STJ04, Swieqi (MT)
(74) Representative: Kaukonen, Juha Veikko

(57) **Abstract**

An arrangement for internal bone support. The arrangement comprises a support device (1) insertable in a cavity (20) of the bone to be supported. The support device (1) includes a frame (2) having first and second end sections (6a, 6b), the frame (2) comprising plurality of longitudinally arranged strips (4). The frame (2) is capable to expand from a reduced configuration to an expanded configuration in a direction perpendicular to the longitudinal centre line (C) of the frame (2).

## Description

### FIELD OF THE INVENTION

The present invention relates to an arrangement for internal bone support.

### BACKGROUND OF THE INVENTION

When a bone fractures, it is immobilized by external or internal fixation in order to give the bone opportunity to build new callus and heal so it can again carry the intended loads. External fixation is accomplished by the use of a plaster cast, or in more complex scenarios, by use of external fixation devices, e.g. scaffolds outside the body that keep the bone in the right position. Internal fixation is typically achieved by means of screws, plates, intramedullary nails, wires, cables, or combination thereof, herein referred to as fracture fixation devices.

For example fractures in the proximal femur are typically treated surgically in an open procedure with large skin incision through layers of fascia and muscle to provide access to the hip thus enabling the above-mentioned means of internal fixation.

An alternative way to treat a proximal femoral fracture is to use joint replacement devices, either total joint replacement or hemiarthroplasty devices. This is an even more complex procedure which is usually chosen if fracture fixation devices would not provide enough stability of the bone due to poor bone quality and insufficient purchase of screws in the bone, or in case there are some other reasons for a more thorough repair, such as osteoarthritis.

If fracture fixation devices are chosen to stabilize the femur, such a fixation is associated with a relatively high failure rate as full immediate load bearing on the joint is not allowed and premature loading will lead to failure of the fixation and will necessitate a reoperation.

Due to the insufficient primary stability of the bone after fracture fixation for the first 6-12 weeks after surgery, patients are forced to rest in bed for extended periods, first in the hospital where the surgery took place and thereafter in a rehabilitation clinic. Depending on the circumstances, the primary care hospital stay is 1-3 weeks and the duration of the secondary rehabilitation care varies from 2-24 weeks.

Currently, about 60% of all hip fractures occur as a result of osteoporosis, a disease that affects bone density and makes them very susceptible to fractures. Even low impact falls may lead to fractures in the hip, typically in the proximal part of femur. Especially in the elderly who represent the majority of osteoporotic patients, this prolonged bed-rest together with their pre-existing other medical pathologies lead to other medical problems which substantially drive up the mortality and morbidity rates.

### BRIEF DESCRIPTION OF THE INVENTION

An object of the present invention is thus to provide an arrangement for internal bone support and a method to overcome the above problems. The objects of the invention are achieved by an arrangement and method which are characterized by what is stated in the independent claims. The preferred embodiments of the invention are disclosed in the dependent claims.

The invention is based on the idea of mechanically immobilize bone by use of a support device which is inserted in a reduced configuration inside the bone and, thereafter, deployed in an expanded configuration inside the bone, the deployed support device comprising strips surrounding the centre line of the support device and intersecting the plane of the fracture at a distance from said centre line.

An advantage of the invention is that the fractured bone can be repositioned and fixed minimally invasive, meaning that only a small access opening is necessary to surgically perform an internal fixation, yet a structure persisting high tensional and torsional forces is achieved.

The technique allows the fixation of even large, load-bearing bones, without any additional implants or support structures.

Furthermore, minimally invasive surgery as opposed to open surgery has clear advantages for the patient and health care providers due to the fact that smaller wounds are produced that heal quicker with less complications and this gives opportunity to release the patients earlier from the hospital. It may also mean less pain for patient and lower cost for health care providers and insurance companies.

Compared to screw and plate fixation arrangements and alike, an advantage of the invention is that loads and stresses inside the bone are more evenly distributed when the invented arrangement is used. This leads to a better initial load-bearing capacity.

An inherent weakness of known screw and plate fixation arrangements is that stresses resulting from load bearing are typically concentrated proximally around the screw head and distally around the screw thread portions, often leading to failures of fixation in these areas. This is especially true when osteoporotic bones are fixed, because such bones possess a very weak internal bony structure where screws cannot be securely inserted with a sufficient purchase.

According to an embodiment of the invention the arrangement comprises further a filling material that is arranged to fill the normally occurring or artificially generated cavity where the expanded support device has been situated, the filling material adhering chemically or physically to the support device. An advantage is that a solid construction supporting the fracture site well is generated as the consequence of the curing or hardening of the filling material.

According to another embodiment of the invention the structure of the frame of the support device is self-expanding. An advantage is that separate means for expansion of the support device are not needed.

According to another embodiment of the invention the support device has a self-drilling frame. An advantage is that there is no need for a separate tool for making a cavity in the bone.

According to another embodiment of the invention the arrangement is at least partly made of a resorbable material. An advantage is that the arrangement will exit gradually the organ system of the patient enabling the bone tissue or other natural tissues filling the space occupied initially by the arrangement, such a feature being particularly beneficial to young patients.

According to another embodiment of the invention the arrangement is at least partly made of a non-resorbable material. An advantage is that the arrangement will permanently support the bone.

According to another embodiment of the invention the support device has protrusions arranged in one or more of the strips and facing away form the longitudinal center line of the device. An advantage is that the support device is able to lock bone pieces around the fracture site to each other. Thus, the risk for an extravasation may be minimized.

According to another embodiment of the invention a sleeve or envelope is arranged around the support device. An advantage is that the risk for an extravasation may be minimized.

According to still another embodiment of the invention the arrangement comprises a support device consisting of a long, thin, spring hard wire, that fills the cavity in the bone through its natural springiness, and the cavity is filled up with a filling material.

### BRIEF DESCRIPTION OF THE DRAWINGS

In the following the invention will be described in greater detail by means of preferred embodiments with reference to the accompanying drawings, in which
Figure 1 is a schematic perspective view of a support device according to the invention in a reduced configuration,
Figure 2 is schematic perspective view of the support device shown in Figure in an expanded configuration,
Figure 3 is a schematic elevation view of a step of a method for implanting a support device according to the invention in femur,
Figure 4 is a schematic elevation view of a second step of a method for implanting a support device according to the invention in femur,
Figure 5 a schematic elevation view of a third step of a method for implanting a support device according to the invention in femur,
Figure 6 is a schematic elevation view of a fourth step of a method for implanting a support device according to the invention in femur,
Figure 7 is a schematic elevation view of a second support device according to the invention in an expanded configuration and arranged in femur,
Figure 8 is a schematic elevation view of a third support device according to the invention in an expanded configuration and arranged in femur,
Figure 9 is a schematic elevation view of a fourth support device according to the invention in an expanded configuration and arranged in femur,
Figure 10 is a schematic elevation view of a fifth support device according to the invention in an expanded configuration and arranged in femur,
Figure 11 is a schematic elevation view of a sixth support device according to the invention in an expanded configuration and arranged in femur,
Figure 12 is a schematic elevation view of a seventh support device according to the invention in an expanded configuration and arranged in femur,
Figure 13 is a schematic elevation view of a eighth support device according to the invention in an expanded configuration and arranged in femur,
Figure 14 is a schematic elevation view of a means for expansion of a support device according to the invention,
Figure 15 is a schematic elevation view of a fifth step of a method for implanting a support device according to the invention in femur,
Figure 16a is a schematic elevation view and Figure 16b cross-sectional view of ninth support device according to the invention in an expanded configuration, and
Figure 17 is a schematic elevation view of tenth support device according to the invention in an expanded configuration and arranged in femur.

For the sake of clarity, the figures show the invention in a simplified manner. Like reference numbers identify like elements.

### DETAILED DESCRIPTION OF THE INVENTION

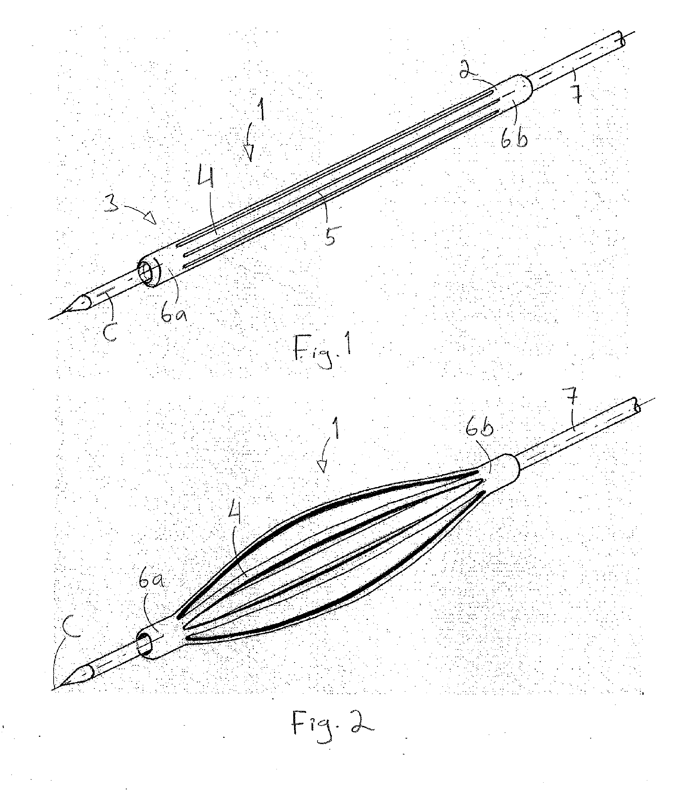

Figure 1 is a schematic perspective view of a support device according to the invention in a reduced configuration, and in Figure 2 in its expanded configuration.

The support device 1 in its reduced configuration comprises an expandable frame 2 that has an oblong tube-like shape having a longitudinal channel 3 therethrough. The cross section of the frame 2 is round and its diameter is constant essentially along its entire length. The imaginary longitudinal centre line of the frame 2 is depicted by reference symbol "C".

It is to be noted here that the cross section of the frame 2 may also be angulated or oval etc.

The frame 2 comprises a number of strips 4 that are arranged longitudinally, i.e. they are arranged at least substantially parallel with the longitudinal centre line C and the channel 3. The number of the strips 4 is preferably 2 to 100, more preferably 4 to 12, most preferably 6 to 8.

A strip 4 is separated from the next one by a slit 5 which extends through the thickness of the wall of the frame 2 from outer surface to the channel 3. The slits 5 may be manufactured to a tube-like blank of the frame 2 by applying, for instance, etching, sawing, milling, laser cutting, grinding or electro-discharge-machining (EDM) methods.

The frame ends at a first end section 6a and a second end section 6b. The strips 4 are connected to each other at said first and second end sections 6a, 6b. In other words, the slits 5 are not extended to the very ends of the blank of which the frame 2 is manufactured but a suitable length of the blank is left unslit.

The frame 2 may be manufactured from a biocompatible grade metal, metal alloy, plastic or plastic composite, or ceramics. Some examples of the materials are stainless steel, magnesium, titanium, Nitinol, tantalum, niobium, carbon-fiber, silicone-carbide, fiberglass, bioglass, Kevlar and PEEK (polyether-ether-ketone).

The material and dimensions of the frame 2 shall be selected so that a sufficiently strong structure supporting the structure of target bone is achieved. The material may be malleable or springy material depending on the principle of the expansion of the frame 2, the principle of which is discussed later in this description.

The frame 2 may stay in the bone as a permanent implant, i.e. the frame 2 may be non-resorbable. According to another embodiment of the invention, the support device 2 is resorbable.

As used herein, the term "resorbable" means that the material is biodegradable, bioerodible or bioabsorbable. By "biodegradable" it is meant that the composition will degrade over time by the action of enzymes, by hydrolyric action and/or by other similar mechanisms in the human body. By "bioerodible," it is meant that the composition will erode or degrade over time due, at least in part, to contact with substances found in the surrounding tissue fluids, cellular action, and the like. By "bioabsorbable," it is meant that the composition will be broken down and absorbed within the human body, for example, by a cell, a tissue, and the like.

The structure of the frame 2 may be self-expanding, i.e. the frame 2 is striving to expand to the expanded configuration shown in Figure 2. This kind of effect may be accomplished in various ways. For example in case of metal-made frame 2, the effect may be achieved by a thermal treatment annealing the already slit frame 2 in its expanded configuration, and then forcing the thermally treated frame 2 in the reduced configuration. Alternatively, the steel frame could be plastically deformed into its expanded position and then forced back into the reduced position until released.

Basically the same kind of heat treatment combined with an orientation through drawing may also be used to produce a self-expanding plasticmade frame 2. The temperature, orientation rate and other parameters of the treatment are selected according to the prerequisites of the material. A drawn product may be a semi-finished product, from which the frame 2 is manufactured by machining or compression moulding, for instance.

The self-expanding frame 2 may be kept in its reduced configuration shown in Figure 1 by a limiter, for instance a sleeve, arranged around the frame 2. The limiter is detachable so that it may be removed on the frame 2. It is to be noted that the limiter is not shown in Figures. Let it be mentioned, however, that it may be a tube that covers the entire frame 2, or it may be just one or more rings that are arranged on about the middle section of the frame 2, or it may have a mesh structure etc. The material of the limiter may be plastic or metal, for example.

According to another embodiment of the invention, the frame 2 is not self-expanding but it is to be expanded by a mechanical force exerted from outside of the support device 1. This kind of mechanically expandable support device 1 may stay in its expanded configuration due to the malleability of the material of the frame 2 or due to maintaining the mechanical force.

Malleability of the material means that the frame 2 made of it is capable to deform into another form or shape due to mechanical force and, furthermore, permanently stay at least substantially in that form or shape after the force has been removed.

Maintaining the mechanical force means that the support device 1 or at least the arrangement comprising the support device 1 includes means for mechanically forcing and changing the form or shape of the frame 2 from its reduced configuration to its expanded configuration in the fracture site. Such a means may include, for instance, an axially acting spring in the middle section of the frame 2. An alternative embodiment of such means comprises a longitudinally arranged screw shaft, a threaded bushing arranged to the first end section 6a or the second end section 6b, and a rotary joint where the screw shaft is attached rotatably to the first end section 6a or the second end section 6b, respectively. The frame 2 is forced to expand as the screw shaft is rotated in relation to the threaded bushing.

The material of the frame 2 may be springy, in which case the frame 2 will recover to or about to its reduced configuration in the case the force is removed.

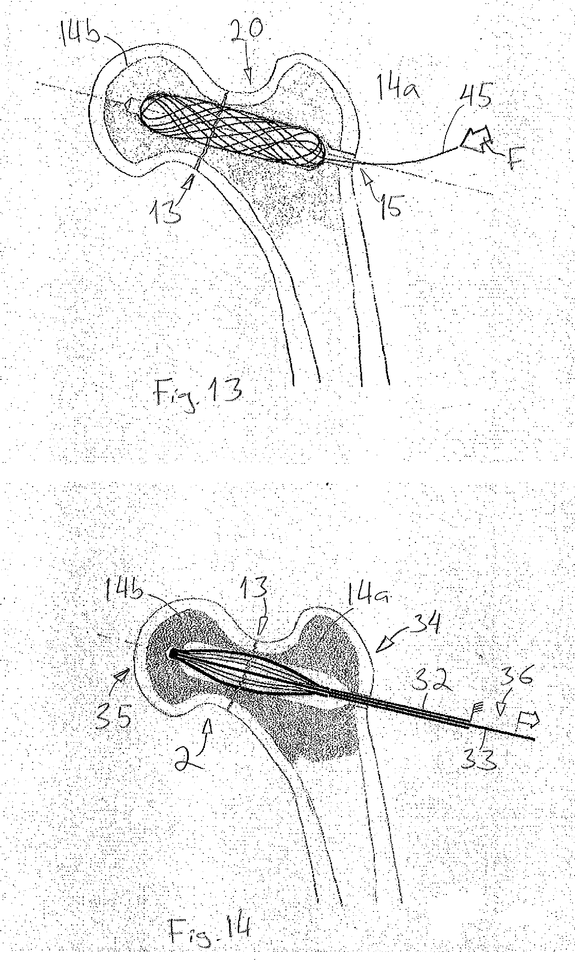

Figure 14 is a schematic elevation view of a means for expansion of the frame 2 shown in Figure 1 or similar tube-like frames. Said means 31 includes a support tube 32 of essentially the same diameter as the frame 2, and a pulling wire 33. An end 34 of the support tube is attached coaxially to the proximal end of the frame 2. The first end 35 of the wire is attached to another end, i.e. the distal end of the frame 2, and the second end 36 of the pulling wire is passed through the support tube 32. The frame 2 will expand in its middle section to a greater diameter when pulling the pulling wire 33 through the support tube 32.

Alternatively, the support tube 32 is pushed and the distal end of the frame 2 is kept about stationary, or the pulling wire 33 is pulled and the support tube 32 is pushed simultaneously.

The means for expansion may, of course, be realized another ways too.

Coming back to Figure 1, the support device 1 is arranged on a K-wire 7. The K-wire 7 is used to guide the support device in the right place in the tissue to be operated. The K-wire 7 is commonly known by persons skilled in the art and therefore it is not discussed more detailed here.

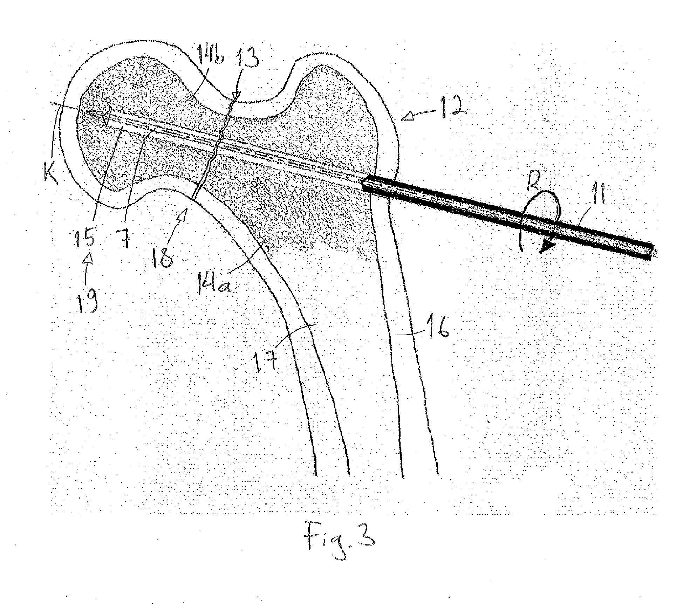

Figure 3 is a schematic elevation view of a step of a method for implanting a support device in the femur according to the invention. The femur or more precisely the proximal part of the femur fractures quite frequently as a consequence of osteoporosis, but other reasons for fracturing are also known.

Nevertheless, any other bones which include a natural channel or cavity or wherein an artificial channel or cavity may be created to nest the stent can be targeted. Some examples of the bones and/or indications are:
- clavicular fractures;
- humeral fractures;
- fractures of the forearm;
- fractures of the metacarpal, metatarsal or phalangeal bones;
- calcaneal and talar fractures;
- tibial fractures;
- fibular fractures;
- pelvic fractures.

The femur 12 has been fractured along a fracture line 13. The fracture line divides the femur 12 into a first and a second fragments 14a, 14b.

Prior to the step shown in Figure 3, the patient has been placed in a supine position so that the surgeon has an access to the operation site. The first and second fragment 14a, 14b have been realigned and the fracture has been fully reduced in a closed setting. These steps have been accomplished without invasive surgery. The reduction of the fracture may be controlled through x-ray or similar detection means.

Then an access point is provided through the skin to the lateral aspect of the proximal femur, at the level of the greater trochanter, i.e. first fragment 14a.

This may be accomplished, for instance, by making a 10 to 20mm incision and using a small retractor.

Next a K-wire 7, diameter of which may be, for instance, 1.5 to 2mm has been entered from the lateral femur through the center of the femoral neck 18 into the femoral head 19.

Figure 3 is showing a step of the method where a pilot hole 15 is drilled through hard cortex or cortical 16 into soft cancellous bone 17 all the way in the femoral head 19. This step is realized by a cannulated drill bit 11, arranged on the K-wire 7, or some other instrument. Such instruments are known per se, so they are not discussed in more detail herein. The imaginary longitudinal centre line of the pilot hole 15 is marked by reference symbol K. The longitudinal centre line K is preferably perpendicular to the fracture line 13 and arranged along the centre line of the affected portion of the bone.

The diameter of the drill is selected according to the requirements of the operation and may be, for instance, in the range of 1-10mm, for example 5mm. The pilot hole 15 is dimensioned so that the support device 1 in its reduced configuration may be inserted therethrough into the cavity 20 without significant effort.

As the pilot hole 15 has been drilled the drill bit 11 is removed from the hole 15 and the K-wire 7 is kept in its place in the hole 15. Alternatively, also the K-wire 7 is removed from the pilot hole 15.

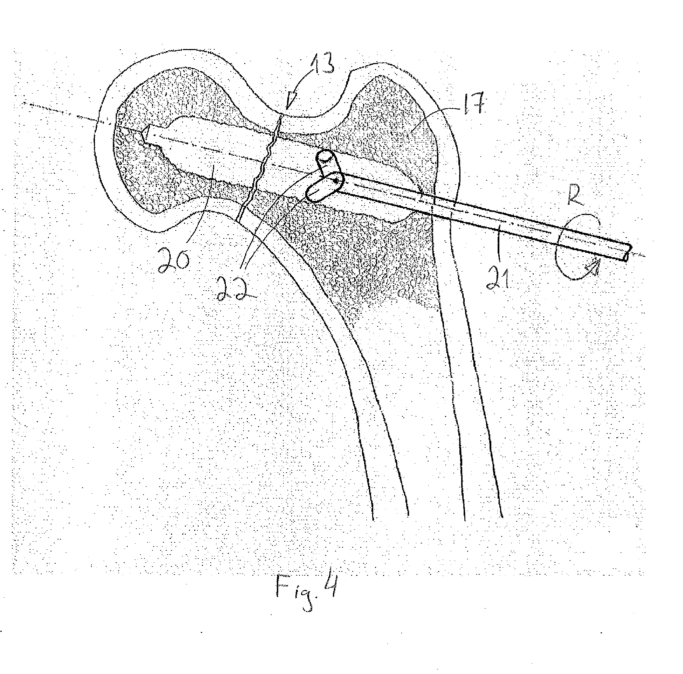

Figure 4 is a schematic elevation view of a second step of a method for implanting a support device according to the invention in femur.

Prior to inserting the support device 1 into the bone, the pilot hole 15 may be enlarged into form of a cavity 20 which for receiving the support device 1 and for expansion it inside the bone. Figure 4 is showing an example of a device 21 for internal enlargement of the pilot hole 15 to produce the cavity 20.

The device 21 is a reamer that is inserted within the pilot hole 15. The reamer comprises at least one blade - here two blades 22 - in its distal end. The blades 22 can be positioned at different distances in relation to the centre line of the device 21. When the device 21 is being inserted in the pilot hole 15, the blades 22 are in a retracted position close to said centre line. They may even be within the distal end of the shaft of the device 21.

The insertion of the device 21 is continued until the distal end of the reamer is deep enough in the pilot hole 15, i.e. the distal end is in area of cancellous bone 17.

After this, the blades 22 are moved outside of the circumference of the distal end of the reamer, i.e. the blades 22 are moved to their outermost position shown in Figure 4. This movement may take place gradually or instantly. The movement of the blades 22 may be driven by the influence of centrifugal force caused by rotation R of the device 21, or there may be a mechanism for it in the device 21.

The device 21 the blades 22 of which are in their outermost position is rotated in the pilot hole 15 by hand or power drill etc. Upon rotation R and axial movement of the device 21 the blades 22 cut the cancellous bone 17 and form the cavity 20 crossing transversally the fracture line 13. The dimensions of the cavity are selected according to the demands of the operation. For example, the diameter of the cavity 20 may be between 15mm and 20mm and the length between 40mm and 100mm. The cutting process and the cavity 20 may be controlled through x-ray control or some other known means. The geometry of the cavity 20 made using the device 21 is rotationally symmetrical, and its cross profile may have constant or alternating diameter.

As the cavity 20 is finished the rotation of the device 21 is stopped and it is withdrawn from the cavity 20 through a part of the pilot hole 15 in the cortex 16. Produced debris is either removed through a suction device or alike, or it is not removed but left in the cavity 20.

Various tools and devices may be used for making the cavity 20 in cancellous bone in the vicinity of the fracture site. In any case the hard bone "shell" being comprised of cortex 16 is preferably intact and not removed.

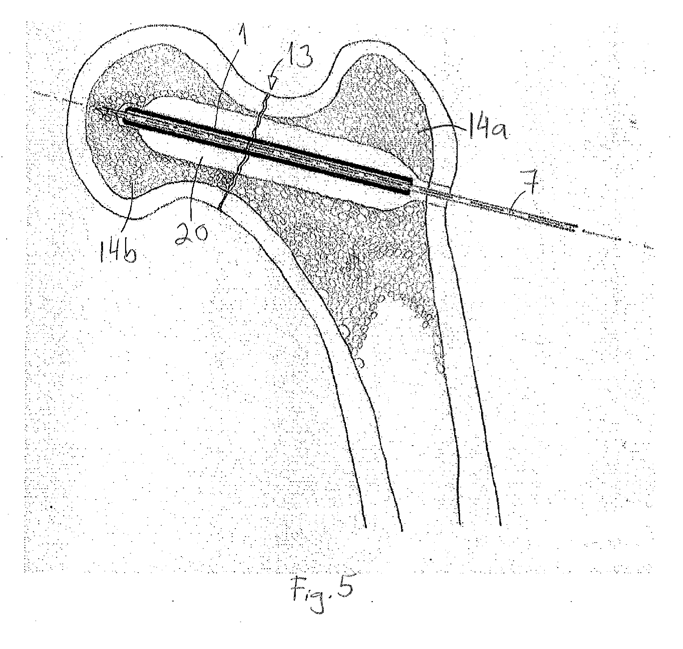

Figure 5 is a schematic elevation view of a third step of a method for implanting a support device according to the invention in femur. The support device 1 of the invention has been inserted in the cavity 20 formed as described above through the pilot hole 15. The support device 1 is in the reduced configuration during the insertion. The K-wire 7 has been used to facilitate the insertion. Nevertheless, the K-wire 7 is not always needed.

The implant 1 may be mounted in the cavity 20 manually or by using instruments known per se. The insertion may be carried out, for instance, as follows: A guide wire or k-wire 7 is introduced in the bone, and the pilot hole is drilled over the wire. Thereafter the pilot hole 15 is enlarged with a special reamer as described above. The support device 1 is then introduced over the very same guide wire or k-wire 7. The guide wire or k-wire 7 directs and centers the support device 1 in the pilot hole 15.

The support device 1 may be pushed with a tube-like instrument over the guide wire or k-wire 7 into the cavity 20. The bone support device 1 may be pre-assembled in an insertion tube or a sleeve that keeps the support device 1 in its reduced position. The insertion tube or sleeve may be pulled back to release the support device 1 after it is positioned in the cavity 20. The insertion tube and/or the sleeve is/are then withdrawn.

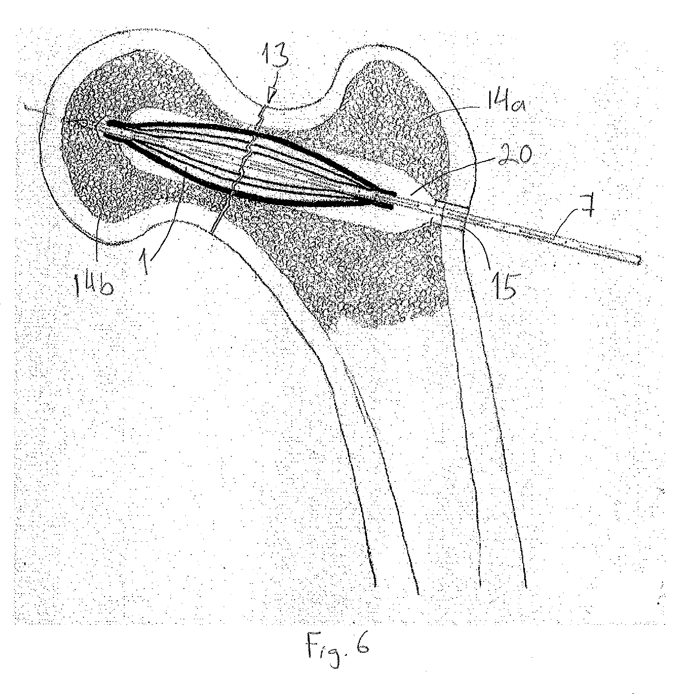

Figure 6 is a schematic elevation view of a fourth step of a method for implanting a support device according to the invention in femur.

The frame 2 of the support device has already been converted into its expanded configuration to such extent that at least some of the strips 4 (as shown in Figures 1 and 2) make a contact with wall of the cavity 20. The frame 2 thus makes a three dimensional structure or scaffold the maximum diameter of which at least substantially corresponds to the inner diameter of the cavity 20.

The expansion of the frame 2 into the expanded configuration may take place several ways, some of which has already discussed in connection with Figures 1 and 2. The support device 1 is preferably radio-opaque, so that its position can be monitored on X-ray by the operating surgeon.

It is to be noted that the artificial cavity 20 is not always created. Instead a naturally occurring cavity such as intramedullary canal of a tubular bone is utilized. Said naturally occurring cavity or hollow may have enlargened due to a disease, e.g. osteporosis.

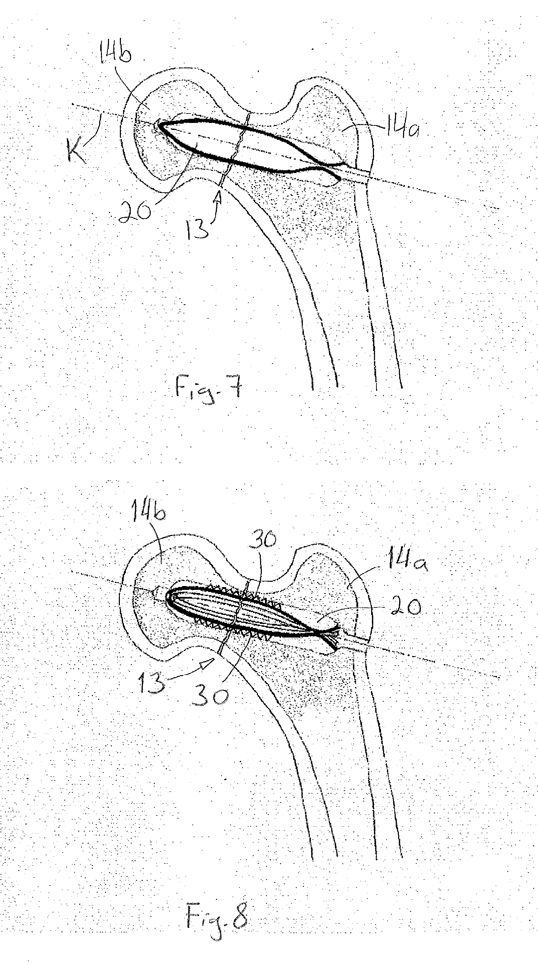

Figure 7 is a schematic elevation view of a second support device according to the invention in an expanded configuration and arranged in femur. The strips 4 are connected to each other at said first and second end sections 6a, 6b, but at the very end of the second end section 6a the strips 4 are arranged to make an extension transversally to the longitudinal centre line C of the device.

The characteristic of this kind of "fishtail spring" device 2 is that it can be forced into an expanded position by a pushing force directed on the fishtail, parallel to the longitudinal centre line C of the device. When the fishtail passes the small orifice of the pilot hole 15 in the lateral femur, it gets a chance to expand laterally so that it is locked in place in the bone. The longitudinal struts are then preferably compressed firmly against the inside wall of the cavity 20, especially at the point where the fracture line 13 crosses the cavity 20.

It is to be noted here that the longitudinal shape of the frame 2 may be non-symmetrical various ways. Its shape may resemble, for instance, a peanut. Said peanut shape may result a better mechanical locking of the fractured bone pieces 14a, 14b and increase the pull-out strength of the device.

Figure 8 is a schematic elevation view of a third support device according to the invention in an expanded configuration and arranged in femur. The support device 1 has teeth or protrusions 30 arranged in one or more strips 4 and facing outwards, i.e. away form the longitudinal centre line of the frame 2. When the support device 1 is expanded in a cavity 20 of the fractured bone, the support device 1 is able to lock the two fractured bone pieces 14a and 14b together. This will facilitate the injection of a filling material in the cavity 20 as the risk will be minimized that portions of the filling material will flow out the cavity 20 through gaps in the fracture 13.

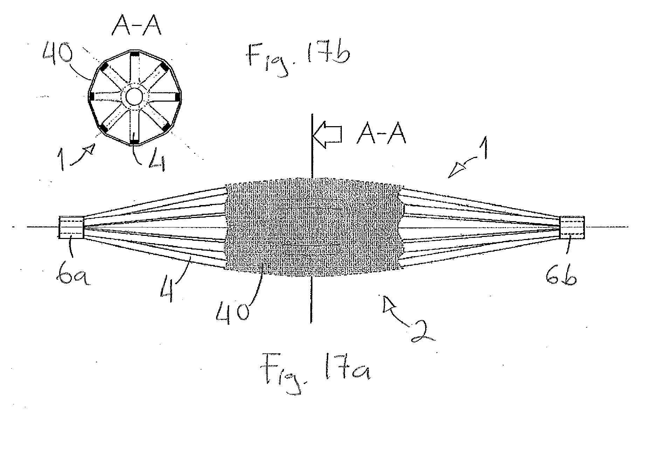

Another way to avoid extravasation is to arrange a permanent or resorbable sleeve or envelope around the support device. Figure 17a is a schematic elevation view of a support device 1 which comprises a sleeve. Figure 17b is a cross sectional view of said support device 1. The support device is shown in its expanded configuration.

The sleeve 40 acts as a seal between the filling material 37 (not shown) and the fracture 13 (not shown). The sleeve 40 is expandable to the size of the nominal diameter of the frame 2 of the support device.

The sleeve 40 may covers only the middle section of the frame 2, i.e. the section which will be in contact with the fracture 13. Alternatively, the length of the sleeve 40 may be approximately same as the frame 2.

The sleeve 40 may be permeable, so that air or low viscosity liquids can penetrate through, but not the filling material high viscosity liquids such as uncured bone cement.

The sleeve 40 may be made of suitable plastic or elastomer.

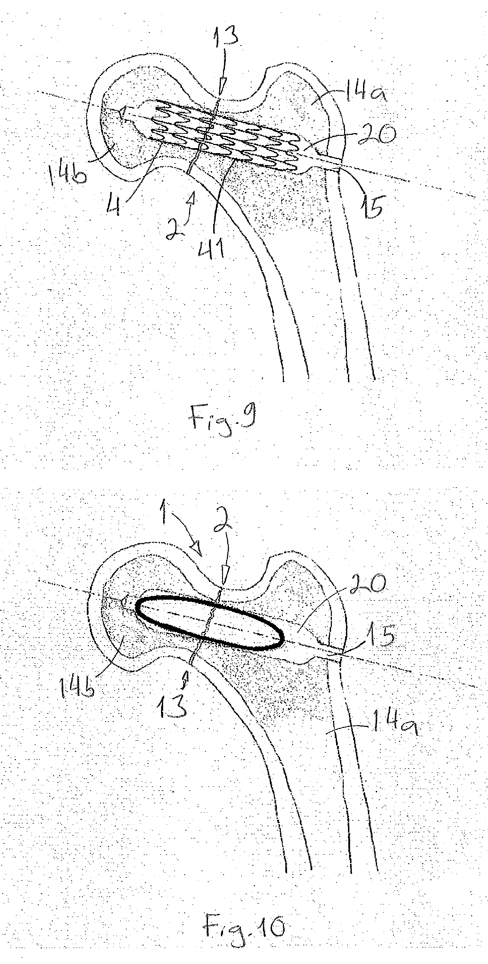

Figure 9 is a schematic elevation view of a fourth support device according to the invention in an expanded configuration and arranged in femur.

The frame 2 is manufactured from a metallic billet in such way that it possesses circumferential structures 41 that can be expanded and will deform permanently, thus rendering the device in an expanded state.

The longitudinal strips 4 are more or less undeformed during expansion, so that shortening of the support device during expansion is minimized. The circumferential structures 41 may be manufactured, for instance, by laser cutting technique. Expansion of the device may be executed through an internal balloon or any other mechanical means that produced radial forces inside the device.

Figure 10 is a schematic elevation view of a fifth support device according to the invention in an expanded configuration and arranged in femur. The expanded device 1 has a ring-shaped configuration. The frame 2 of the device consists mainly of longitudinal strips 4 that are relatively thin and made preferably from spring-steel or ceramic.

The strips 4 are so thin that the device can be reduced to fit through a small orifice like the pilot hole 15 in the femur. The frame has not necessarily to be reduced or held down during implantation of the device 1 as the frame 2 can be pushed through a small hole by a pushing rod on the front side of the ring facing the orifice, i.e. the side of the frame 2 which is first introduced in the pilot hole 15. It is to be noted that the pushing rod is not shown in the figure.

When the push force is applied, the ring automatically reduces its size to fit through the orifice. As the ring arrives in the cavity 20, i.e. the hole becomes wider, the ring spring will again expand such that it presses against the internal wall of the cavity 20. The ring spring may be cut from a larger spring-hard metal tube as well it may be manufactured from a bundle of ceramic fibers. This embodiment is particularly simple and cost-effective to produce.

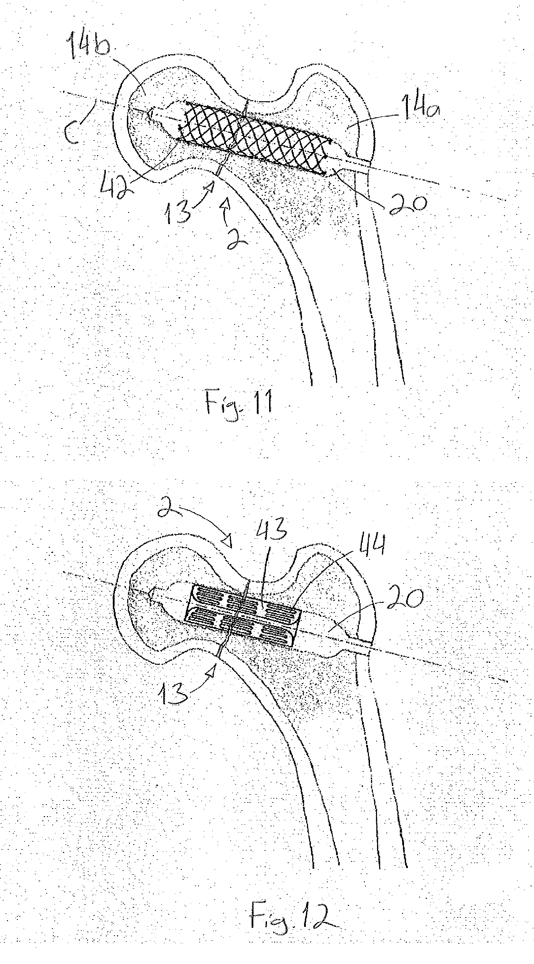

Figure 11 is a schematic elevation view of a sixth support device according to the invention in an expanded configuration and arranged in femur.

The frame 2 of the device comprises of a number of metal or ceramic wires 42 that establish a tubular, expandable structure in which said wires 42 surrounds helically the longitudinal centre line C of the device.

The tubular structure is preferable self-expanding and it is held down in a reduced configuration during insertion of the device. When the device expands inside the cavity 20, it is significantly shortened, but the struts are still covering the fracture line 13.

This kind of configuration may be produced in a cost-effective manner by using a braiding-machine. For example a continuous tubular structure can be manufactured that is then cut into a number of frames 2.

Figure 12 is a schematic elevation view of a seventh support device according to the invention in an expanded configuration and arranged in femur. In this embodiment the frame 2 comprises a thin metallic sheet or foil 43. The foil 43 is made of a springy material such as spring steel.

As the device 1 is in its reduced configuration, the foil 43 is rolled up tightly and kept in the configuration by a withholding sleeve or other means. The foil 43 expands upon release of the sleeve back into its stress-free flat configuration. In that it resembles a coil spring which is used in watches to keep the clockwork going. As the foil 43 is released in the cavity 20, it presses against the wall of said cavity 20.

The foil 43 has openings 44 that a) make it easier to roll into its reduced configuration, and b) make it possible for the filling material to penetrate through the foil 43 and cover both surfaces of the foil 43 so that it becomes embedded in the filling material.

The device could be manufactured e.g. from A 304 or 316L stainless steel foil or sheet which is delivered spring hard. This sheet may be between 0.01 and 0.1 mm thick and it may be etched, laser-cut or machined by EDM techniques, for instance.

Figure 13 is a schematic elevation view of a seventh support device according to the invention in an expanded configuration and arranged in femur.

The frame 2 of the support device is comprised of a long, thin, spring hard metallic wire 45 which comprises first and second end sections and a middle section therebetween. The wire 45 is fed F through the pilot hole 15 in the cavity 20.

The wire 45 is inserted in the cavity 20 through, for instance, a tube-like feeder device, wherein it is forced to a fairly straight shape. As soon as the wire hits the end wall of the cavity 20, it is bent to be apposed against the wall of bone canal, i.e. in a curved and meandering shape through its natural springiness. By delivering more footage the so generated wire or mesh structure will cover most of the inside wall of the bone cavity 20 and produces an expanded frame configuration. Thus, a reinforcing structure can be produced inside the bone by inserting a suitable length of wire in the cavity 20. The frame 2 is then filled up with a filling material as described earlier in this description.

The wire may also be made from a ceramic material, such as resorbable or non-resorbable glass. The diameter of such glass wire is pereferably 5-100 micron.

According to an embodiment of the invention, the wire is impregnated with cyano-acrylate or some other fast curing biocompatible adhesive, prior to the injection of the filling material. Cyano-acrylate will populate the interspaces of the wire and it will form a seal in the fracture line 13 to avoid extravasation. The cyano-acrylate may also create a strong bond between the wire and the bone.

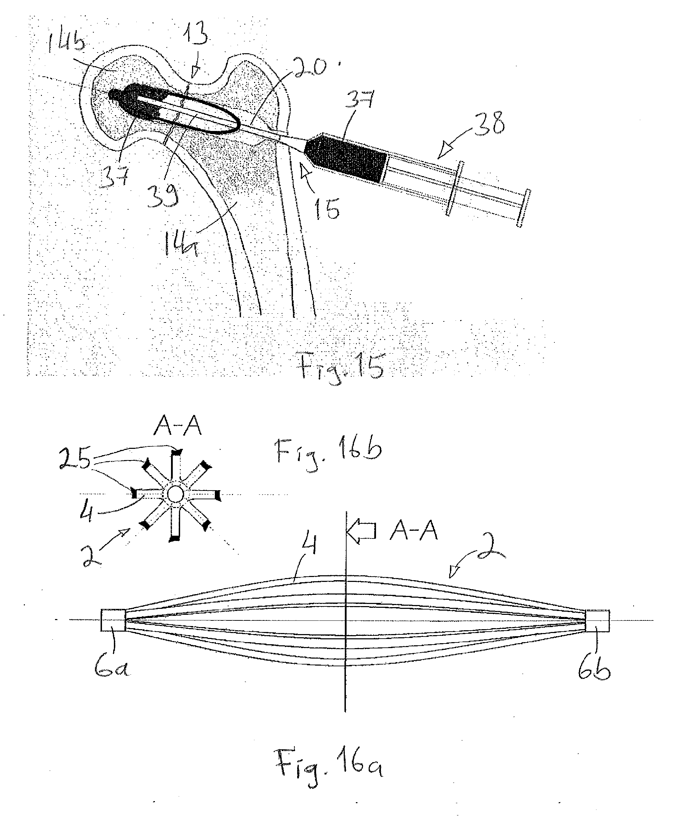

Figure 15 is a schematic elevation view of a fifth step of a method for implanting a support device according to the invention in femur.

The expanded support device 1 still in the cavity 20, a filling material 37 is injected in order to fill the free space in the cavity 20. The filling material 37 is selected so that it adheres chemically, i.e. by chemical bonds, or by physical bonds, or by both to the support device 1. The filling material 37 preferably adheres also to the bony tissue to further increase the fixation strength in the fracture site.

A sufficient amount of filling material 37 is injected in the cavity 20 by using an injection device, such as a syringe 38 shown in Figure 15, and a suitable nozzle 39 that fits in the pilot hole 15 in the cortex. The cavity 20 and preferably also the pilot hole 15 in the cortex will be completely filled with the filling material 37 whereupon the frame 2 is sufficiently embedded in the filling material 37. This may be controlled by X-ray means.

The filling material 37 may be of polymeric or ceramic nature or mixture thereof. It may be resorbable or non-resorbable material. Some examples of the filling material 37 are:
- acrylic polymers, such as methacrylates, BISGMA, HEMA, TEGDMA, UDMA, PMMA
- glass ionomer composites;
- light curable PLAs ;
- polyanhidrides or other curable polymers;
- polyurethanes;
- cyano-acrylates;
- calcium-phosphates;
- calcium-sulphates;
   or composition thereof.

The filling material 37 may comprise one or more fillers. Potential fillers of polymeric filling materials are, for instance:
- TCP (tricalciumphosphate);
- nano-TCP;
- glass beads, diameter of which is preferably 0.1 - 1.0mm;
- magnesium spheres or other metallic spheres or powder;
- glass fibers;
- silicone carbide;
- carbon fibers;
- polymer spheres such as PMMA beads;
- polymer fibers;
- radiopaque fillers, such as BaSO₄;
- titanium oxide;
   or composition thereof.

A solid composition construction comprising the frame 2 and the filing material 37 will be generated when the filling material 37 has set, i.e. cured or hardened. This construction aligns the pieces 14a, 14b of bone and then builds a load-bearing structure inside the bone. The loads and stresses acting on the fracture site may be borne mainly by the frame 2, whereas the filling material 37 distributes the stresses to and supports the frame 2. The support device-filling material construction possesses very high bending, torsional, shear, tensile and compressive strengths due to the presence of the support device 1. The construction will allow for immediate load bearing in the bone and total immobilization which will lead to less pain as "a stable fracture is pain free fracture".

Known bone cements used to embed femoral stems in femoral canals do not possess sufficient bending strength to allow the use for fracture fixation. The combination of the support device 1 and filling material 37 possesses sufficient strength because the support device 1 acts as a reinforcing scaffold inside the filling material 37, the structure of which is analogical to steel reinforcements in concrete in construction technology.

The support device-filling material construction may stay in the bone as a permanent implant, i.e. the construction may be non-resorbable. According to another embodiment of the invention the support device 1 is non-resorbable whereas the filling material 37 is resorbable. This means that the support device 1 will stay permanently in the bony tissue but the filling material is replaced by new bony tissue that fills the cavity 20 in the course of time. According to still another embodiment of the invention, the whole construction may also be resorbable, i.e. both the support device 1 and the filling material 37 are replaced by new bony tissue.

As soon as the injection device is removed and the filling material 37 cured or hardened, the surgical access to the operational area can be closed with a suitable closing means, e.g. suture material.

The patient is put at rest preferably for a period not substantially exceeding the setting time of the filling material 37 and/or the clearance of the body of any anaesthesia or pain relief pharmaceuticals used, usually about at least 12 hours before loading the fracture.

Figure 16a is a schematic elevation view and Figure 16b cross-sectional view of another support device according to the invention in an expanded configuration. The support device here has a self-drilling frame 2.

The frame 2 comprises eight longitudinally arranged strips 4. Each of the strips 4 comprises an outer edge constituting a cutting blade 25. The strips 4 are preferably made of metal that is capable to be sharpened, or the blade may be constructed from a separate element that has been attached to the strip 4.

The self-drilling frame 2 is preferably self-expanding and it is forced to rotate using an attachable driver. The driver may itself be cannulated and attached to, for instance, a slow turning power drill. As the frame 2 is turned it gradually scrapes off cancellous bone from the external wall of the pilot hole 15 while it gradually expands. This way, the pilot hole 15 is enlarged slowly until a desired cavity 20 has been formed into the bony tissue. The size of the cavity 20 may be detected by x-ray or by observing dimensional change of the frame 2 in its longitudinal direction.

When using the frame 2 as a cutting tool, the frame 2 will be nicely embedded in the bone before the filling material 37 is injected, thus producing a tight connection between the support device-filling material construction and the host bone. Therefore, the self-drilling frame 2 is preferably left in place in the cavity 20 and the filling material 37 is injected into the cavity 20 as already discussed.

In another embodiment of the invention the strip 4 comprises two outer edges with blade 25 arranged such that first outer edge may cut when the frame 2 is rotating clockwise and second outer edge when the frame 2 is rotating counter clockwise. This kind of self cutting frame 2 may be rotated, for instance, in an oscillating way in two directions.

Using the self-drilling frame 2 one can combine the afore-mentioned steps of forming the cavity 20 and expansion of the frame 2, thus avoiding the separate step of forming the cavity 20 with an extra tool.

It will be obvious to a person skilled in the art that, as the technology advances, the inventive concept can be implemented in various ways. The invention and its embodiments are not limited to the examples described above but may vary within the scope of the claims.

## Claims

1. An arrangement for internal bone support, **characterized in that**
the arrangement comprises a support device (1) insertable in a cavity (20) of the bone to be supported,
the support device (1) including a frame (2) having first and second end sections (6a, 6b),
the frame (2) comprising plurality of longitudinally arranged strips (4),
the frame (2) having a capacity to expand from a reduced configuration to an expanded configuration in a direction perpendicular to the longitudinal centre line (C) of the frame (2).

2. The arrangement as claimed in claim 1, **characterized in that** the expansion of the support device (1) is arranged to take place by bending the middle sections of the strips (4) outwards while the ends of the strips (4) are arranged to remain interconnected at the first and the second end (6a, 6b) of the frame.

3. The arrangement as claimed in claim 1, **characterized in that** it further comprises a filling material (37) that is arranged to fill space specified by the cavity (20) and the expanded frame (2) therein, the filling material being capable to set in said space.

4. The arrangement as claimed in claim 3, **characterized in that** the filling material (37) is arranged to adhere chemically and/or physically to the expanded frame (2).

5. The arrangement as claimed in claim 3 or 4, **characterized in that** the filling material (37) comprises one or more materials selected from acrylic polymers, such as methacrylates, BISGMA, HEMA, TEGDMA, UDMA, glass ionomer composites, light curable PLAs, cyano-acrylates, or curable ceramic compositions such as calcium-sulphate or calcium-phosphate.

6. The arrangement as claimed in any of claims 3 - 5, **characterized in that** the filling material (37) comprises one or more fillers selected from TCP (tricalciumphosphate), calcium-sulphate, glass beads and/or powder, magnesium spheres and/or powder, glass fibers, polymer spheres, polymer fibers, radiopaque fillers.

7. The arrangement as claimed in any of the preceding claims, **characterized in that** the structure of the frame (2) is self-expanding.

8. The arrangement as claimed in any of the preceding claims, **characterized in that** the frame (2) comprises at least one protrusion (30) arranged in one or more of the strips (4) and facing away form the longitudinal centre line (C) of the frame.

9. The arrangement as claimed in any of the preceding claims, **characterized in that** it comprises a sleeve (40) arranged around the frame (2) for minimizing an extravasation.

10. The arrangement as claimed in any of the preceding claims, **characterized in that** the strips (4) comprise an outer edge constituting a cutting blade (25) capable to cut the bony tissue for making the cavity (20).

11. The arrangement as claimed in any of claims 1 - 9, **characterized in that** the frame (2) consists of a wire (42) that is arranged to expand into the expanded configuration through its natural relaxation into a curved and meandering shape through its natural springiness in free space of the cavity (20).

12. The arrangement as claimed in any of claims 1 - 10, **characterized in that** the device (1) has a ring-shaped form in its expanded configuration and that the frame (2) consists mainly of longitudinal strips (4) that are made from spring-steel or ceramic.

13. The arrangement as claimed in any of claims 1 - 10, **characterized in that** the frame (2) comprises of a number of wires (42) that establish a tubular, expandable structure in which said wires (42) are arranged to surround helically the longitudinal centre line (C) of the device.

14. The arrangement as claimed in any of claims 1 - 10, **characterized in that** the frame (2) comprises a sheet or foil (43) made of a springy material and that the stress-free configuration of said sheet or foil (43) is flat.

15. The arrangement as claimed in any of claims 1 - 10, **characterized in that** the strips (4) are connected to each other at the first and second end sections (6a, 6b) and arranged to make an extension transversally to the longitudinal centre line (C) of the device at the very end of the second end section (6a).
